# EUROPEAN PATENT SPECIFICATION

(11) **EP 0 980 687 B1**
(45) Date of publication and mention of the grant of the patent: **03.03.2004**
(21) Application number: 98115744.9
(22) Date of filing: 20.08.1998
(51) Int. Cl.: A61M 5/145

(54) **Portable automatic syringe device**
Tragbare, automatische Spritzenvorrichtung
Dispositif de seringue automatique portable

(43) Date of publication of application: 23.02.2000
(60) Divisional application: 01122747.7
(73) Proprietor: Sooil Development Co., Ltd., Seoul (KR)
(72) Inventor: Choi, Soo Bong, Chungju-shi, Chungcheongbuk-do (KR)
(74) Representative: Kuhnen & Wacker

(56) References cited:
- US-A- 4 351 335
- US-A- 4 585 439
- US-A- 4 978 335

## Description

### BACKGROUND OF THE INVENTION

### Field of the Invention

The present invention relates to a portable automatic syringe device enabling an injection of liquid medicine for a prolonged time according to claim 1.

More particularly the present invention relates to a portable automatic syringe device having a configuration including a separable rotating shaft adapted to provide a drive force to a piston included in the automatic syringe device so that the rotating shaft can be separated, along with the piston, from a housing of the syringe device upon re-filling a syringe of the syringe device with a liquid medicine, and set in position in the housing after the re--filling of the liquid medicine.

### Description of the Prior Art

From US-A-4,585,439 a portable automatic syringe device is known. With this known syringe device a driver sleeve is pivoted in the casing of a unit, which is driven by a motor controlled by a control device and supplied by a battery. A threaded rod forming the piston rod of an injection ampule is placed into the driver sleeve in a non-rotary but longitudinally shiftable fashion. A nut provided with an external toothing is rigidly held on the rear end of the injection ampule in the casing. A connecting part provided with a catheter receives the output piece of the injection ampule and is screwed to the casing so that the exchangeable injection ampule is completely surrounded by the casing and the connecting part and the output piece, resting close to the connecting part, of the injection ampule communicates with the catheter. The threaded rod is placed into the piston of the injection ampule with the nut being screwed on and is placed into the casing together with the injection ampule. When the driver sleeve is driven, the piston rod is moved longitudinally, since the nut is held tightly in the casing adjacent to the rear end of the injection ampule. The unit guarantees a reliable advance of the piston rod even with a low torque of the driving device and has only a length as required by the injection ampule and the necessary piston rod length.

From US 4,978,335 A an infusion pump is known, which comprises:
a housing, mounting means on the housing for at syringe having a plunger, means including a motor connected to the plunger for driving the plunger at a preselected rate, a computer connected to the motor for controlling the operation of the plunger, said computer being capable of receiving data relating to patients dosage requirements and converting that data to an output that controls said motor to drive said plunger in accordance with received data,
a reader for machine-readable computer data connected to the computer, a syringe having a plunger and having machine-readable data mounted on the syringe and containing data relating to a patient's dosage requirement, the reader, upon reading the data, controlling the operation of the motor and hence the dispensing of medicant in the syringe, said syringe being mounted by mounting means, to enable the reader to read a data and program the computer for a particular patient's requirements.

Automatic syringe devices, which enable an injection of liquid medicine for a prolonged time, are also well known. Typically, known automatic syringe devices have a configuration in which a push means for pushing a syringe piston is coupled to a housing receiving an injection syringe. For example, such automatic syringe devices are disclosed in Japanese Utility Model Laid-open Publication No. Sho. 52-3292 and U.S. Patent No. 4,417,889). The syringe device disclosed in Japanese Utility Model Laid-open Publication No. Sho. 52-3292 has inconvenience in carrying it because it has an injector mounted outside a basic case, thereby requiring a double case structure. In order to solve such a disadvantage, an automatic syringe device requiring no double case structure has been proposed, as in the above-mentioned U.S. Patent No. 4,417,889. Figs. 1 and 2 illustrate a control circuit and a structure of the automatic syringe device disclosed in U.S. Patent No. 4,417,889, respectively.

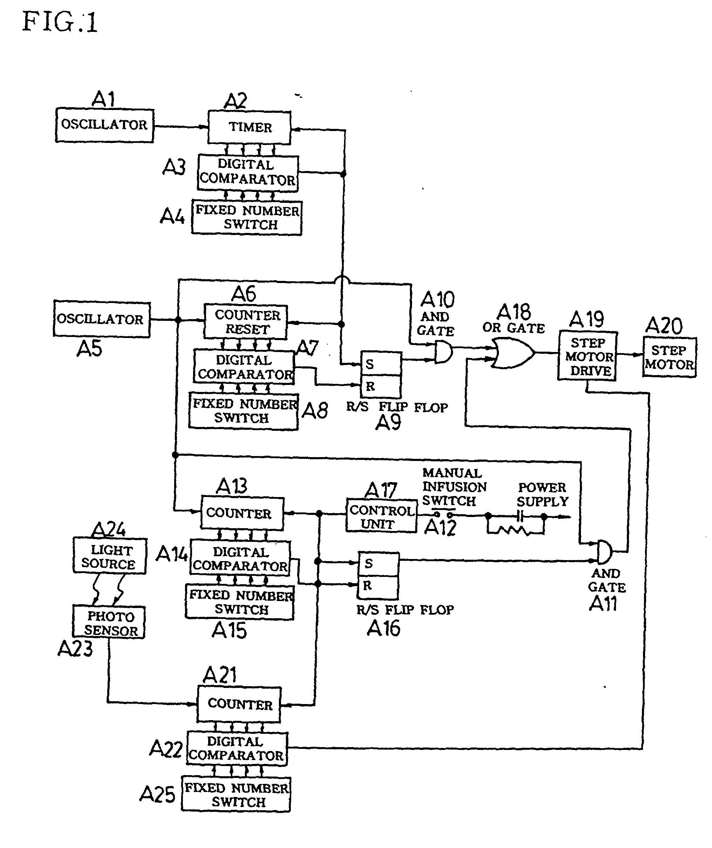

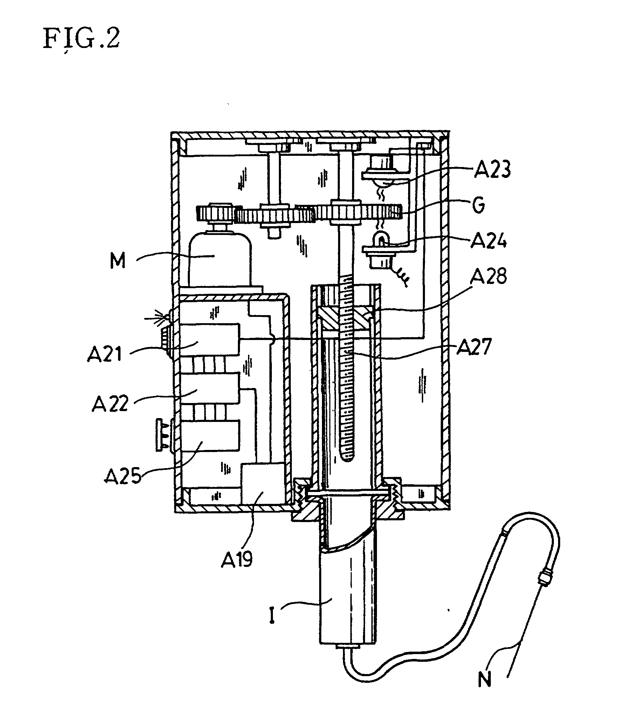

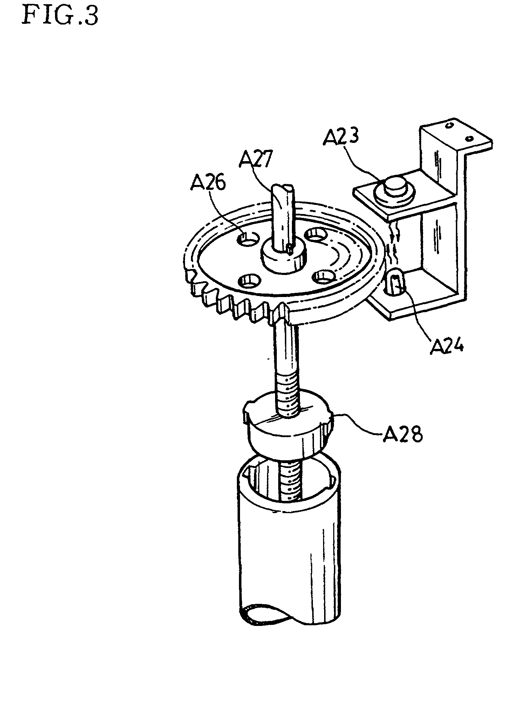

Referring to Fig. 1, the output of an oscillator A1 is coupled to a timer A2 which is, in turn, coupled at its output to a digital comparator A3. The digital comparator A3 also receives an output from a fixed number switch A4. The output of the digital comparator A3 is connected to a counter A6 and an R/S flip-flop A9. Another oscillator A5 is also provided which has an output coupled to counters A6 and A13, and AND gates A10 and A11. The flip-flop A9 is reset by an output from a digital comparator A7. Another R/S flip-flop A16 is also provided which is reset by an output from a digital comparator A14 coupled to the counter A13. A control unit A17 is also coupled to the counter A13. The control unit A17 serves to activate the counter A13 in accordance with an operation of a manual infusion switch A12. The control unit A17 applies its output to the counters A13 and A16. The output from the control unit A17 is also sent to a counter A21. The output of the counter A21 is coupled to a digital comparator A22 which is, in turn, coupled to a step motor driver A19 for driving a step motor A20. The output of the flip-flop A16 is coupled to one input of the AND gate A11, which is also coupled at the other input thereof to the oscillator A5. The output of the AND gate A11 is coupled to one input of an OR gate A18. Fixed number switches A15 and A25 are connected to the digital comparators A14 and A22, respectively. Each of the fixed number switches A4, A8, A15, and A25 has five protruding insert bars and serves to provide a reference value for an associated one of the digital comparators A3, A7, A14, and A22. A light source A24 and a photo sensor A23 are coupled to the counter A21 in order to provide sensing results thereof to the counter A21, respectively. Referring to Figs. 2 and 3, the arrangements of the light source A24 and photo sensor A23 are illustrated. As shown in Figs. 2 and 3, the light source A24 and photo sensor A23 are arranged in such a fashion that they face each other while being vertically spaced from each other. A gear plate, which is included in a gear mechanism G, is interposed between the light source A24 and photo sensor A23. The gear plate has a plurality of through holes A26 uniformly spaced from one another in a circumferential direction, as shown in Fig. 3. The gear plate is fixedly fitted around a gear shaft A27 having a screw portion. A piston plate A28 is threadedly coupled to the gear shaft A27 in the form of a nut in such a fashion that it slides along the screw portion of the gear shaft A27 when the gear shaft A27 rotates. The rotation of the gear shaft A is carried out by a drive force from the motor A20 transmitted via the gear mechanism G. The driving of the motor A20 is controlled by the operations of the counter A21, digital comparator A22, switch A25, and motor drive A19. All the above mentioned elements of the syringe device are received in a housing. In particular, the light source A24 and photo sensor A23 are fixedly mounted at an upper portion of the housing by means of a bracket fixed to the housing. In this syringe device, a liquid medicine, such as insulin, contained in a syringe I is outwardly injected through an injection needle N connected to the syringe I, by a slide movement of the piston plate A28. In such a syringe device, however, the housing and syringe I thereof are exposed to ambient air. As a result, moisture and water are likely to penetrate into the syringe device. For this reason, there is inconvenience in that if the user desires to take a shower while the syringe is in place, then the housing should be contained in a separate sealing case.

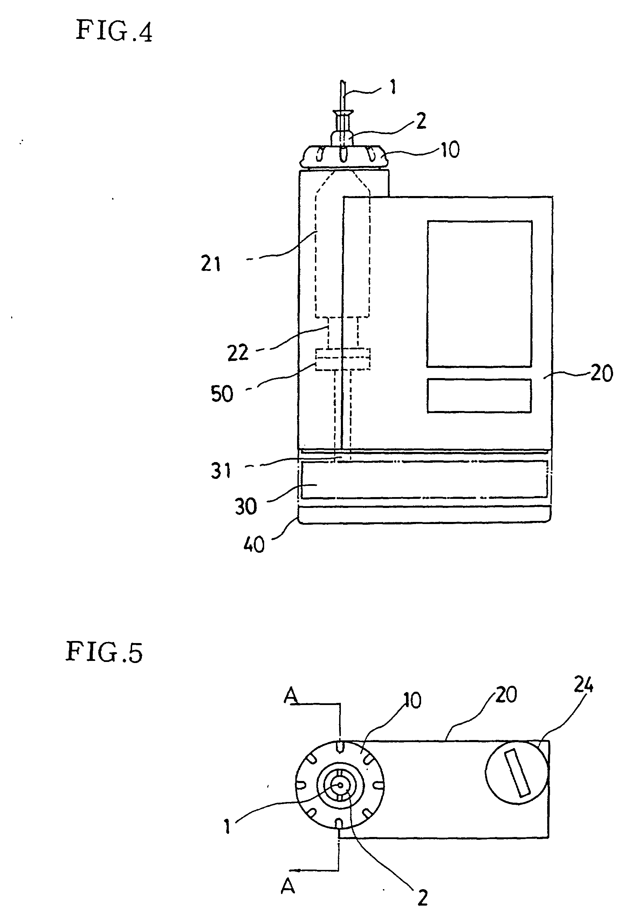

In order to solve such a problem, a sealable syringe device has been proposed by the applicant. Such a sealable syringe device is illustrated in Fig. 4 which is a front view. Referring to Fig. 4, the syringe device includes a cover 10 sealably coupled to the upper end of a housing 10, and a bottom cover 40 sealably coupled to the lower end of the housing 10. A connector 2, to which a feeding tube is integrally connected, is threadedly coupled to the cover 10. The connector 2 communicates with a syringe 21 received in the housing 10. A piston 22 is slidably fitted in the syringe 21. A liquid medicine to be syringed is contained in the syringe 21. A power transmission means 30 is mounted on the bottom surface of the housing 20. The power transmission means 30 has a rotating shaft 31 to which a disc type push means 50 is threadedly coupled. The disc type push means 50 moves vertically by a rotation of the rotating shaft 31, thereby vertically moving the piston 22.

Referring to Fig. 5, which is a plan view of Fig. 4, the cover 10, to which the connector 2 connected with the feeding tube 1 is connected, is arranged on the left portion of the upper surface of the housing 20. A battery cover 24 is arranged on the right portion of the upper surface of the housing 20.

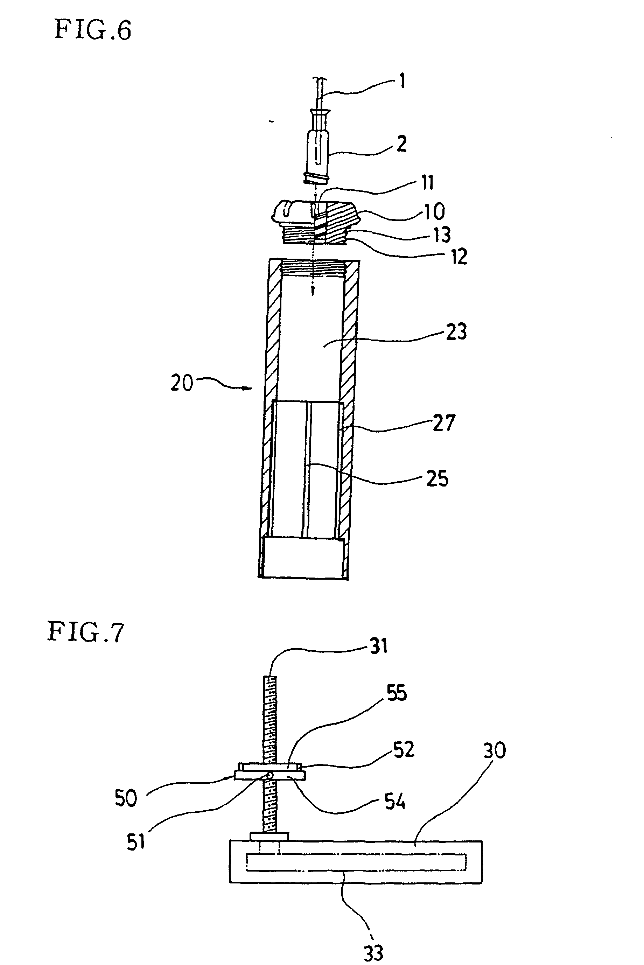

Fig. 6 is a cross-sectional view taken along the line A-A of Fig. 5. As shown in Fig. 6, the cover 10 is centrally provided with a threaded hole 11 in which the connector 2 is threadedly fitted at its lower end. The cover 10 is also provided at its lower end with a bolt portion 12 threadedly fitted in the upper end of the housing 20. A packing 13 is fitted around the bolt portion 12 of the cover 10 between the lower end of the cover 10 and the upper end of the housing 20. A syringe receiving chamber 23 is defined in the interior of the housing 20. The push means 50 is fitted in the lower end of the housing 20 in such a fashion that it slides vertically in the housing 20. The housing 20 is also formed at its inner surface with a vertical push means guide groove 25 adapted to guide a vertical movement of the push means 50 and vertical piston guide grooves 27 adapted to guide a vertical movement of the piston 22.

Fig. 7 shows a detailed configuration of the push means 50 threadedly coupled to the rotating shaft 31 of the power transmission means 30, along with a detailed configuration of the power transmission means 30. As shown in Fig. 7, the push means 50 includes a lower disc 54 threadedly coupled to the rotating shaft 31 in such a fashion that it slides vertically along the rotating shaft 31. The lower disc 54 is provided at its periphery with a guide protrusion 51 engaged in the guide groove 25 of the housing 20 and adapted to guide the vertical movement of the lower disc 54. The push means 50 also includes an upper disc 55 integrally formed with the lower disc 54. The upper disc 55 is provided at its periphery with an engagement means 52. The upper disc 55 is fitted in a sleeve plate 26 fixed to the lower end of the piston 22 in such a manner that its engagement means 52 engages with a mating engagement means formed on the inner peripheral surface of the sleeve plate 26. The sleeve plate 26 is also provided at its outer peripheral surface with protrusions engaging with the guide grooves 27 respectively. The power transmission means 30 includes a reduction mechanism 33 for transmitting the rotating force of a motor (not shown) to the rotating shaft 31 in a speed-reduced manner.

In order to use the syringe device having the above mentioned configuration, the piston 22, which is in a state separated from the housing 20, is first fitted in the syringe 21 which is also in a state separated from the housing 20, in such a manner that it is completely inserted into the syringe 21. In this state, a disposable injection needle (not shown) is fitted onto the tip 21-1 of the syringe 21. Thereafter, the injection needle is penetrated into the interior of a phial through the plug of the phial. In this state, the piston 22 is pulled to suck a liquid medicine (for example, insulin) contained in the phial into the syringe 21.

The piston 22, which is in a state fitted in the syringe 21 containing the liquid medicine, is then inserted into the syringe receiving chamber 23 of the housing 20 in such a manner that it is seated on the push means 50. Subsequently, the cover 10 is threadedly coupled to the upper end of the housing 23. The connector 2 is then threadedly fastened to the cover 10. As the connector 2 is threadedly fastened to the cover 10, it is fitted onto the syringe tip 21-1. Thus, the syringe 21 is maintained in a sealed state in the housing 20. When the motor (not shown) drives under the above condition, the push means 50 moves upwardly, thereby upwardly pushing the piston 22. As a result, the liquid medicine contained in the syringe 21 is outwardly injected from the syringe 21. At this time, the upward movement of the push means 50 is accurately carried out because its guide protrusion 51 engages with the guide groove 25. Since the engagement means 52 of the push means 50 engages with the mating engagement means of the sleeve plate 26 integrally formed with the lower end of the piston 22, the upward movement of the piston 22 is also accurately carried out.

In such a syringe device, it is necessary to set the initial height or vertical position of the lower disc 54 of the push means 50 every time the syringe 21 filled with a liquid medicine is inserted into the housing 20, in order to allow the piston 22 to be accurately seated on the upper disc 55 of the push means 50. However, it is difficult to accurately set a desired initial vertical position of the lower disc 54.

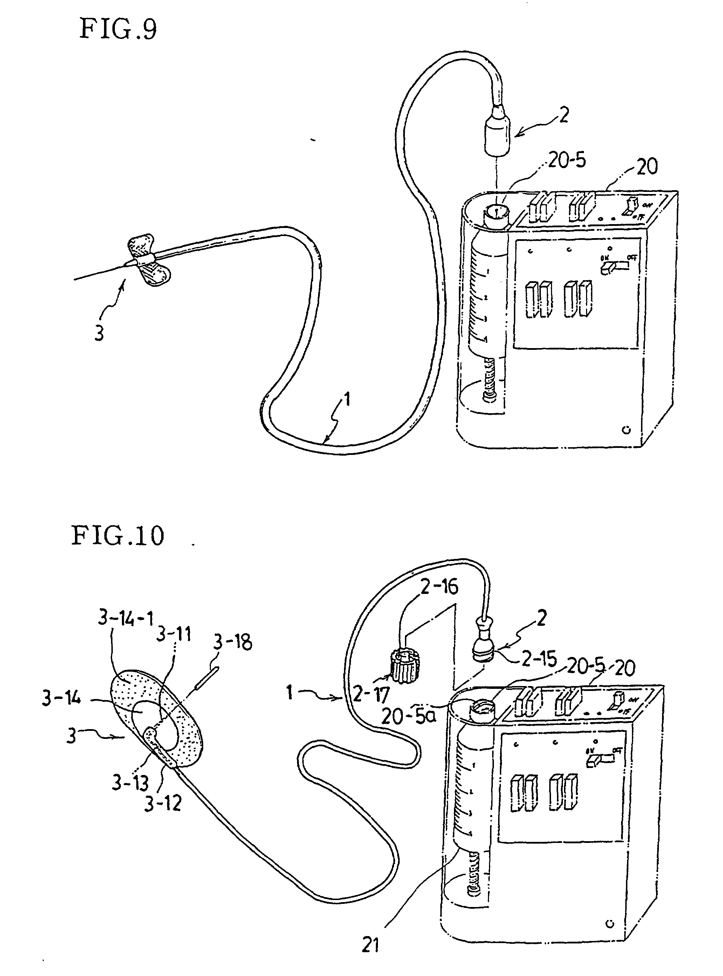

Meanwhile, Fig. 9 illustrates an example of a conventional injection needle unit used for portable automatic syringe devices enabling a prolonged injection of a liquid medicine. As shown in Fig. 9, the injection needle unit includes a feeding tube 1, a "-" shaped straight injection needle member (called a "straight butterfly-shaped injection needle") 3 connected to one end of the feeding tube 1, and a connector 2 connected to the other end of the feeding tube 1.

In order to use such an injection needle unit, the user himself slantly penetrates the injection needle member 3 into the subcutaneous tissue while observing the penetration of the injection needle member 3 with the naked eye. The reason why the user observes the penetration of the injection needle member 3 with the naked eye is because the injection needle member 3 has a straight shape. However, such an observation is very uncomfortable.

Furthermore, when the straight butterfly-shaped injection needle member 3 penetrates the subcutaneous tissue of the user on a slanted angle, its tip may be easily blocked by the subcutaneous tissue because the subcutaneous tissue is a multilayer tissue. As a result, the above mentioned conventional injection needle unit has a drawback in that it is difficult to smoothly inject the liquid medicine, namely, insulin.

The straight butterfly-shaped injection needle member 3 is also likely to move in the subcutaneous tissue of the user because it penetrates the subcutaneous tissue of the user on a slanted angle. In this case, the subcutaneous tissue may be damaged. In severe cases, blood may flow out of the subcutaneous tissue. The user may also feel a severe pain.

As mentioned above, the conventional injection needle unit has a drawback in that it is difficult to smoothly inject insulin because the injection needle member 3, which penetrates the subcutaneous tissue of the user on a slanted angle, may be easily blocked at its tip by the subcutaneous tissue. To this end, the feeding tube of such a conventional injection needle unit inevitably has an increased diameter. However, such a feeding tube having an increased diameter results in a possibility of an excessive insulin injection. In addition, this may result in wastage of expensive insulin. For instance, where it is desired to inject insulin into the user using an automatic syringe device equipped with the above mentioned injection needle unit, it is necessary to completely vent air existing in the feeding tube 1 and injection needle member 3 before penetrating the injection needle member 3 into the subcutaneous tissue of the user. To this end, insulin, which is contained in the syringe device, is outwardly discharged through the feeding tube 1 and injection needle member 3, thereby venting air. In this case, a large amount of insulin is wasted where the conventional injection needle unit having the diameter-increased feeding tube is used.

The use of such a diameter-increased feeding tube also results in an increase in the manufacturing costs.

In the case of the injection needle unit illustrated in Fig. 9, its connector 2 is simply fitted onto a connector portion 20-5 of the syringe device housing 20. For this reason, the connector 2 may be incidentally separated from the connector portion 20-5 of the housing 20.

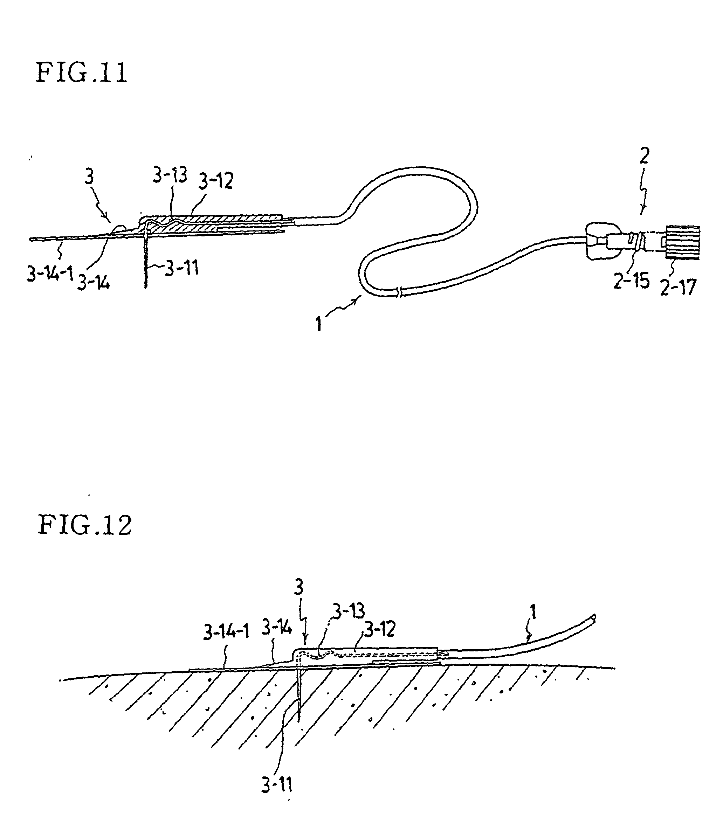

In order to solve this problem, an injection needle unit has been proposed which has a configuration capable of preventing a separation of its connector. Such an injection needle unit is illustrated in Figs. 10 and 11, respectively.

As shown in Figs. 10 and 11, the injection needle unit includes a feeding tube 1, an injection needle member 3 connected to one end of the feeding tube 1, and a connector 2 connected to the other end of the feeding tube 1.

In the case of the injection needle unit shown in Figs. 10 and 11, the injection needle member 3 has an "L" shaped injection needle 3-11. This injection needle 3-11 has a first portion, namely, a horizontal portion, fitted in a connecting rib 3-12 integrally formed with one end of the feeding tube 1, and a second portion, namely, a vertical portion, provided with a needle tip. The injection needle 3-11 is provided with a curved portion 3-13 at its horizontal portion fitted in the connecting rib 3-12, as shown in Fig. 11. A depressing member 3-14 is integrally formed with the connecting rib 3-12 in such a fashion that the injection needle 3-11 protrudes perpendicularly from the depressing member 3-14. The depressing member 3-14 is depressed against the skin of the user upon penetrating the injection needle member 3 into the subcutaneous tissue. A bacterial infection prevention member 3-14-1, which is made of a disinfected nonwoven fabric, is attached to the surface of the depressing member 3-14 which comes into contact with the skin of the user upon penetrating the injection needle unit 3 into the subcutaneous tissue. The feeding tube 1 of Figs. 10 and 11 has a reduced diameter and an increased length, as compared to that of Fig. 9. The connector 2, which is connected to the other end of the feeding tube 1, has a male thread 2-15. The connector 2 is protected by a protection cap 2-17 which has a.female thread 2-16 threadedly coupled to the male thread 2-15 of the connector 2. In use, the connector 2 is threadedly coupled to a connector portion 20-5 of a housing 20 included in an automatic insulin syringe device. The connector portion 20-5 of the housing 20 has a female thread 20-5a threadedly coupled to the male thread 2-15 of the connector 2. In Fig. 10, the reference numeral "3-18" denotes a needle protection cap.

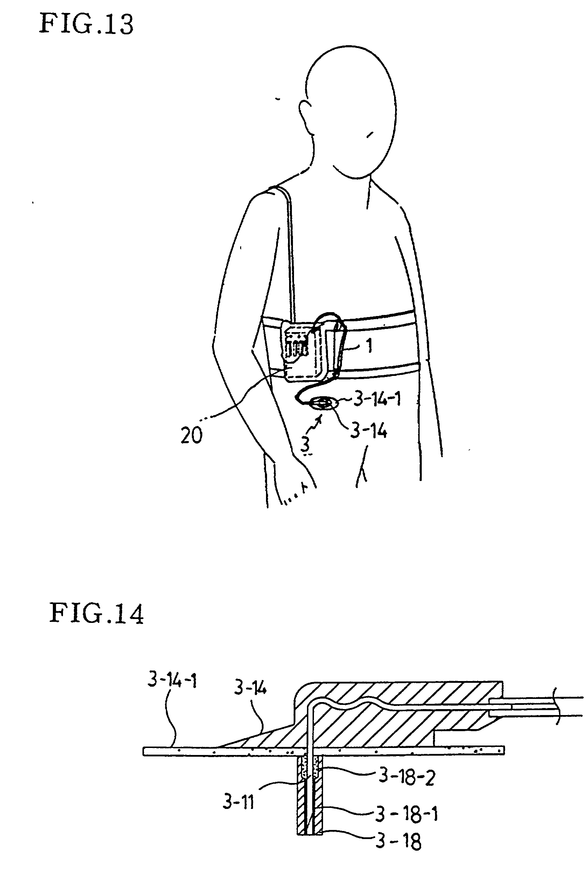

Where it is desired to inject insulin contained in the automatic insulin syringe device using the above mentioned injection needle unit, the protection cap 2-17 is first separated from the connector 2, which is, in turn, threadedly coupled to the connector portion 20-5 of the housing 20. Thereafter, the needle protection cap 3-18 is separated from the injection needle 3-11. The user then penetrates the injection needle 3-11 into the subcutaneous tissue while depressing the depressing member 3-14 against the skin by fingers. At this time, the injection needle 3-11 penetrates vertically into the subcutaneous tissue of the user because it has an "L" shape. Accordingly, the user can carry out the penetration of the injection needle 3-11 instantaneously without any observation with the naked eye. Therefore, the user feels little pain upon penetrating the injection needle 3-11 into the subcutaneous tissue. By virtue of such a configuration of the injection needle unit 3, the automatic insulin syringe device can be conveniently used, as shown in Fig. 13. Since the injection needle 3-11 penetrates vertically into the subcutaneous tissue of the user by virtue of its "L" shape, there is no phenomenon that the injection needle 3-11 is blocked at its tip by the subcutaneous tissue of the user. Thus, the injection of insulin is smoothly carried out. Accordingly, the feeding tube can have a reduced diameter and an increased length. Since the feeding tube 1 has a reduced diameter, it is possible to minimize the wastage of insulin occurring upon venting air existing in the feeding tube 1 and injection needle 3-11 and to reduce the manufacturing costs. Since the feeding tube 1 also has an increased length, it is possible to extend the range of the position of the injection needle 3-11 on the body of the user. Accordingly, it is possible to achieve convenience in use. Since the bacterial infection prevention member 3-14-1, which is made of a disinfected nonwoven fabric, is attached to the depressing member 3-14, it is. possible to prevent the depressing member 3-14 from coming into direct contact with the skin of the user upon penetrating the injection needle unit 3 into the subcutaneous tissue. Accordingly, it is possible to prevent the user from being infected.

Since the injection needle 3-11 penetrates vertically into the subcutaneous tissue of the user by virtue of its "L" shape, as mentioned above, it hardly moves in the subcutaneous tissue even when an external force is applied thereto. Accordingly, there is no damage of the subcutaneous tissue. Of course, there is no phenomenon that the blood flows out of the subcutaneous tissue. The user also does not feel any pain.

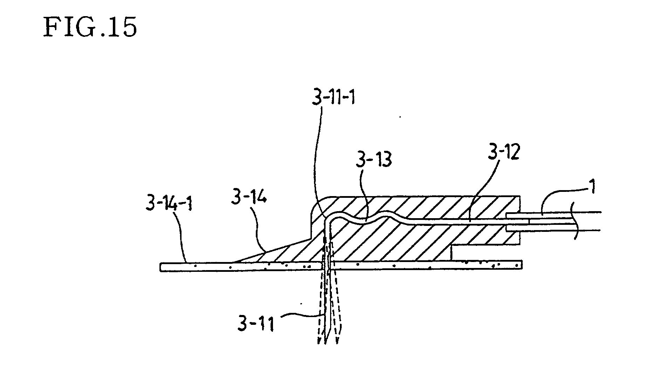

In the case of the injection needle unit mentioned above, the needle protection cap 3-18 is used which has a configuration as shown in Fig. 14. The needle protection cap 3-18 has a needle tip receiving hole including a smaller diameter portion 3-18-1 with the same diameter as the injection needle 3-11 and a larger diameter portion 3-18-2 with a diameter larger than the diameter of the injection needle 3-11. Since the needle protection cap 3-18 has such a configuration, there is a problem in that it is difficult to separate the needle protection cap 3-18 from the injection needle 3-11 because of the small diameter of the diameter portion 3-18-1. As a result, the injection needle 3-11 may be damaged. Since the smaller diameter portion 3-18-1 has a small diameter, a capillarity phenomenon may occur between the inner surface of the needle protection cap 3-18 and the outer surface of the injection needle 3-11 when the liquid medicine is outwardly discharged from the injection needle 3-11 to vent air existing in the feeding tube 1 and injection needle 3-11. In this case, a part of the discharged liquid medicine is absorbed in the bacterial infection prevention member 3-14-1, thereby causing the user to be uncomfortable. The injection needle 3-11 has a sharp bent portion 3-11-1 between the vertical and horizontal portions thereof due to its "L"-shaped structure. This sharp bent portion 3-11-1 of the injection needle 3-11 may be subjected to excessive stress when the user moves excessively during injection. For instance, when the needle tip of the injection needle 3-11 moves from a position indicated by the solid line of Fig. 15 to a position indicated by the phantom line of Fig. 15 as the user exercises or conducts hard work, or due to other reasons, the sharp bent portion 3-11-1 of the injection needle 3-11 may be subjected to excessive stress. In this case, the injection needle 3-11 may be broken. For this reason, the reliability of the above mentioned injection needle unit is degraded.

### SUMMARY OF THE INVENTION

The present invention has been made in view of the above mentioned problems involved in conventional automatic syringe devices and conventional injection needle units, and an object of the invention is to provide a portable automatic syringe device having a configuration including a separable rotating shaft adapted to provide a drive force to a piston included in the automatic syringe device so that the rotating shaft can be separated, along with the piston, from a housing of the syringe device upon re-filling a syringe of the syringe device with a liquid medicine, and set in position in the housing, after the re-filling of the liquid medicine, while observing the setting operation with the naked eye.

According to the present invention the above object is solved by the features of claim 1.

Improved embodiments of the inventive portable automatic syringe device result from the subclaims.

In accordance with one aspect, the present invention provides a portable automatic syringe device comprising a housing, a syringe separably received in the housing and contained with a liquid medicine, an injection needle unit coupled to the housing in such a fashion that it communicates with the syringe, a piston slidably fitted in the syringe and separably received in the housing, the piston being vertically movable in the syringe to inject the liquid medicine into the injection needle unit, a vertical rotating shaft having a screw extending throughout the length thereof, push means threadedly coupled to the screw of the rotating shaft in such a fashion that it moves along the rotating shaft when the rotating shaft rotates, the push means being adapted to move the piston by the movement thereof, and power transmission means fixedly installed in the housing and adapted to transmit a drive force generated from power generating means to the rotating shaft, further comprising: coupling means for separably coupling said rotating shaft to said power transmission means, so that the rotating shaft is separable from said housing.

### BRIEF DESCRIPTION OF THE DRAWINGS

Other objects and aspects of the invention will become apparent from the following description of embodiments with reference to the accompanying drawings in which:
Fig. 1 is a block diagram illustrating a control circuit used in a conventional automatic syringe device;
Fig. 2 is a cross-sectional view illustrating a structure of the automatic syringe device shown in Fig. 1;
Fig. 3 is a perspective view illustrating the installation of a photo sensor in the automatic syringe device shown in Fig. 1;
Fig. 4 is a front view illustrating another conventional automatic syringe device;
Fig. 5 is a plan view of Fig. 4;
Fig. 6 is a cross-sectional view taken along the line A-A of Fig. 2;
Fig. 7 is a view illustrating a conventional power transmission means;

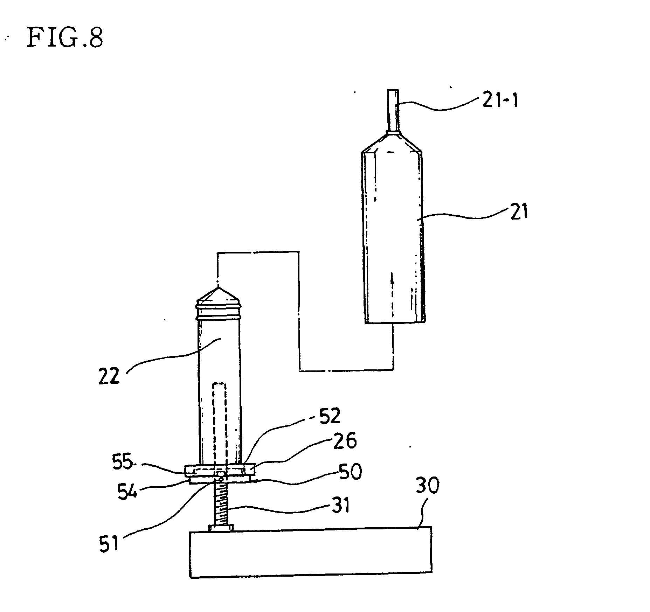

Fig. 8 is an exploded view illustrating a conventional push means;
Fig. 9 is a perspective view illustrating an example of a conventional injection needle unit used for portable automatic syringe devices;
Fig. 10 is a perspective view illustrating another conventional injection needle unit;
Fig. 11 is a partially-broken plan view illustrating the injection needle unit of Fig. 10;
Fig. 12 is an enlarged view illustrating a using condition of the injection needle unit of Fig. 10;
Fig. 13 is a perspective view illustrating a using condition of the injection needle unit of Fig. 10;
Fig. 14 is an enlarged view illustrating a part of the injection needle unit of Fig. 10;
Fig. 15 is a view illustrating a drawback occurring when the injection needle unit of Fig. 10 is used;
Fig. 16 is a perspective view illustrating a portable automatic syringe device according to an embodiment of the present invention;
Fig. 17 is a plan view of Fig. 16;
Fig. 18 is a plan view similar to Fig. 17, but eliminating a cover;
Fig. 19 is a cross-sectional view taken along the line B - B of Fig. 18;
Fig. 20 is an enlarged perspective view illustrating a part of the automatic syringe device of Fig. 16;
Fig. 21 is an enlarged perspective view illustrating a part of the automatic syringe device of Fig. 16;
Fig. 22 is a cross-sectional view illustrating the coupled state of the elements of Fig. 21; and
Fig. 23 is an enlarged cross-sectional view illustrating a reset button installed in accordance with the present invention;

### DESCRIPTION OF THE PREFERRED EMBODIMENTS

### First Type Portable Automatic Syringe Device Enabling Prolonged Injection of Liquid Medicine

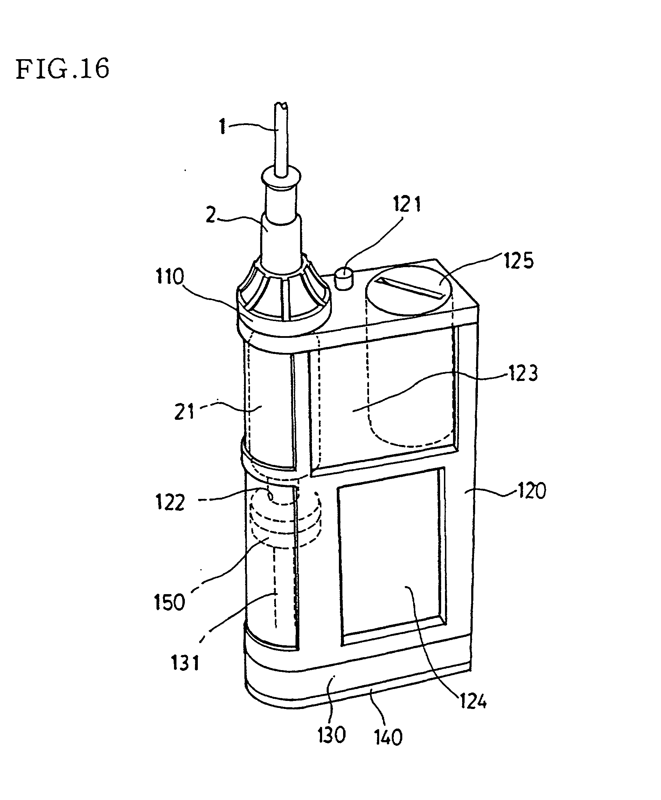

Fig. 16 is a perspective view illustrating a portable automatic syringe device according to an embodiment of the present invention. As shown in Fig. 16, the syringe device includes a housing 120, a syringe 21 separably received in the housing 120, a piston 122 slidably fitted in the syringe 21 and separably received in the housing 120, a piston push means 150 received in the housing 120 and adapted to vertically move the piston 122, a power transmission means 130 received in the housing 120 and adapted to generate a drive force, and a rotating shaft 131 received in the housing 120 and adapted to drive the piston push means 150 by the drive force transmitted from the power transmission means 130. The syringe device also includes an injection needle unit (in Fig. 16, only its feeding tube 1 and connector 2 are shown). The injection needle unit is connected to the housing 120 by means of a cover 110 which is sealably coupled to the upper end of the housing 120 at one side of the housing 120. A manipulation panel 123 is also installed on the housing 120. The manipulation panel 123 is electrically connected to a control circuit (not shown) installed in the housing 120 to control the power transmission means 130. A display 124 such as an LCD is also installed on the housing 120 in order to display the entire condition of the syringe device. At the other side of the housing 120, a battery cover 125 is separably coupled to the upper end of the housing 120 in order to carry a battery in the housing 120. A reset button 121 is also installed on the housing 120 to generate a reset signal for the control circuit. In Fig. 16, the reference numeral "140" is a bottom cover.

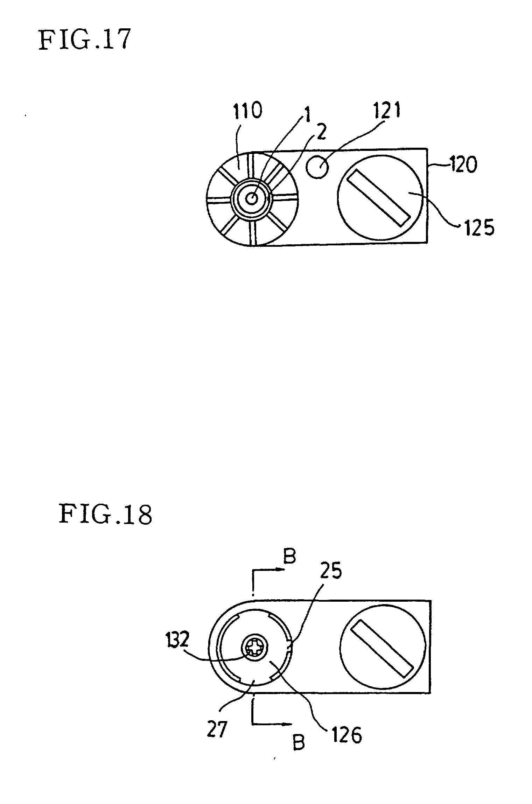

Referring to Fig. 17, which is a plan view of Fig. 16, the cover 110 and battery cover 125 are arranged at opposite sides of the upper wall of the housing 120, respectively. The reset button 121 is arranged on the upper wall of the housing 120 between the covers 110 and 125.

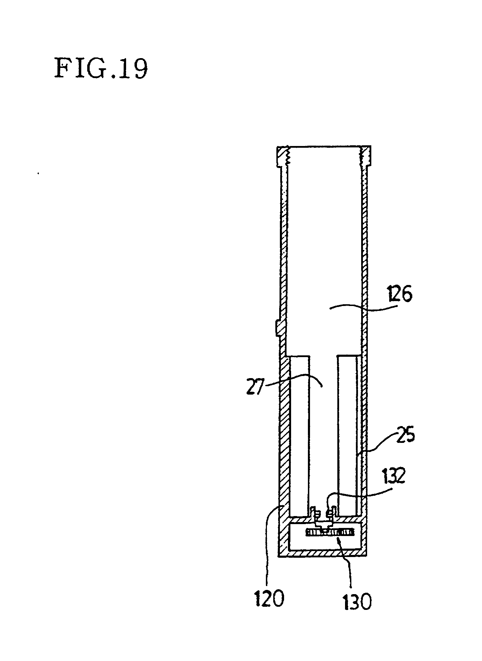

Fig. 18 is a plan view similar to Fig. 17, but eliminating the cover 110. Fig. 18 illustrates the inner construction of the housing 120 in which the piston 122 and piston push means 150 are received. Fig. 19 is a cross-sectional view taken along the line B - B of Fig. 18. As shown in Fig. 19, the housing 120 has a syringe receiving chamber 126 defined in the interior of the housing 120. At the lower end of the syringe receiving chamber 126, the housing 120 has a hollow support portion in which a coupling member 132 coupled to the power transmission means 130 is rotatably fitted. The housing 120 is also formed, at its inner surface defining the syringe receiving chamber 126, with a vertical push means guide groove 25 adapted to guide a vertical movement of the push means 150 and vertical piston guide grooves 27 adapted to guide a vertical movement of the piston 122.

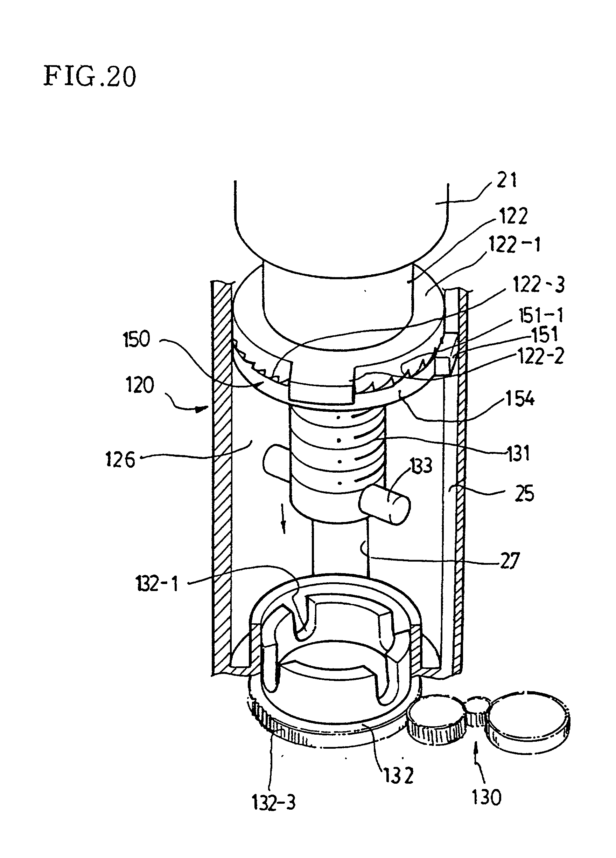

Fig. 20 is an enlarged perspective view illustrating the configuration of the coupling member 132 to which the rotating shaft 131 is coupled. As mentioned above, the coupling member 132 is rotatably fitted in the hollow support portion of the housing 120 at the lower end of the syringe receiving chamber 126. As shown in Fig. 20, the coupling member 132 has a cross groove 132-1 in which a horizontal engaging pin 133 coupled to the lower end of the rotating shaft 131 is separably engaged. A gear 132-3 is also integrally formed with the coupling member 132. The gear 132-3 engages with an output gear of the power transmission means 130. Both ends of the engaging pin 133 are protruded from opposite sides of the lower end of the rotating shaft 131, respectively. By such a configuration, the coupling member 132 rotates by a drive force transmitted from the power transmission means 130 via the gear 132-3, thereby causing the rotating shaft 131 to rotate.

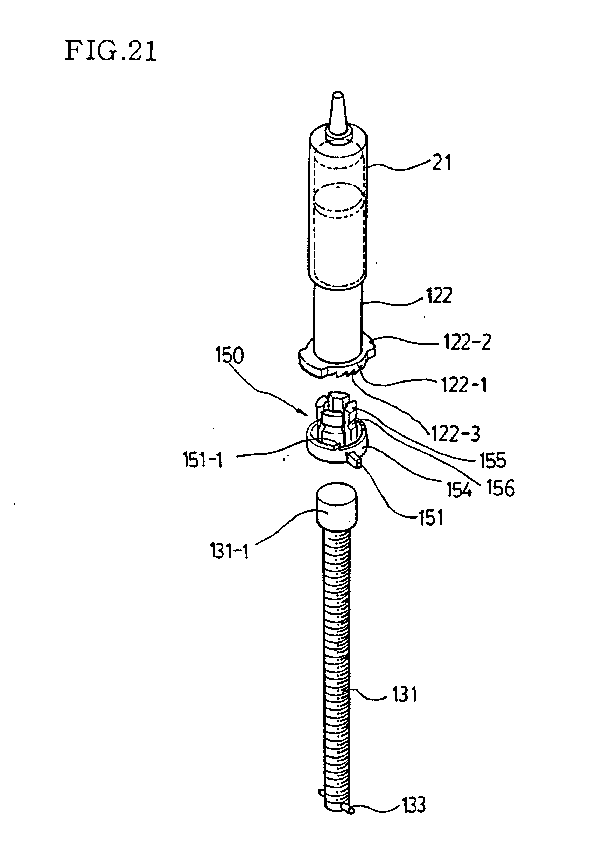

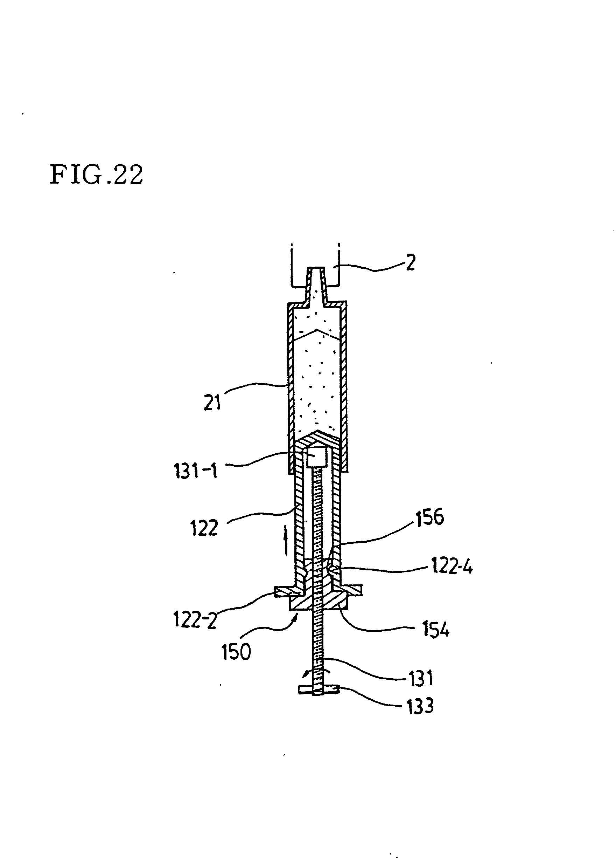

Fig. 21 is an exploded perspective view illustrating the rotating shaft 131, piston push means 150, piston 122, and syringe 21 separated from one another. Fig. 22 is a cross-sectional view illustrating the coupled state of the elements of Fig. 21. As shown in Figs. 21 and 22, the rotating shaft 131 has a screw extending throughout the length thereof. A cap type head 131-1 is threadedly coupled to the upper end of the rotating shaft 131. The piston push means 150 is threadedly coupled to the rotating shaft 131 in such a fashion that it moves vertically along the rotating shaft 131. The piston push means 150 includes a push plate 154 threadedly coupled to the rotating shaft 131 in the form of a nut in such a fashion that it slides vertically along the rotating shaft 131. The push plate 154 is provided at its periphery with a radially-extending guide protrusion 151 engaged in the guide groove 25 of the housing 120 and adapted to guide the vertical movement of the push plate 154. The push plate 154 is also provided at its upper end with engaging protrusions 151-1. The piston push means 150 also includes a fitting member 155 extending upwardly from the push plate 154. The fitting member 155 is fitted into the lower end of the piston 122 which is open. An annular snap ring groove 156 is formed on the outer surface of the fitting member 155. The piston 122 has, at its lower portion, a snap ring 122-4 engaging with the snap ring groove 156. The piston 122 is also provided at its lower end with a radially-extending flange 122-1. A pair of radially-extending protrusions 122-2 are formed on the periphery of the flange 122-1. When the piston 122 is received in the syringe receiving chamber 126, the protrusions 122-2 engage with the guide grooves 27 of the housing 120, respectively, thereby guiding the vertical movement of the piston 122. A plurality of engaging grooves 122-3 are formed on the lower surface of the flange 122-1. When the piston push means 150 is fitted into the lower end of the piston 122, the engaging protrusions 151-1 thereof engage with optional ones of the engaging grooves 122-3 of the piston 122. In the illustrated case, the engaging grooves 122-3 have a small pitch to have the form of gear teeth whereas the protrusions 151-1 have a large pitch. In this case, it is possible to achieve an easy assembling process. In order to achieve an easier assembling process, the guide protrusions 122-2 of the piston 122 may be eliminated, thereby eliminating the reference position of the piston 122 upon assembling the piston 122. Of course, the provision of the guide protrusions 122-2 provides an advantage in that the piston 122 operates more accurately.

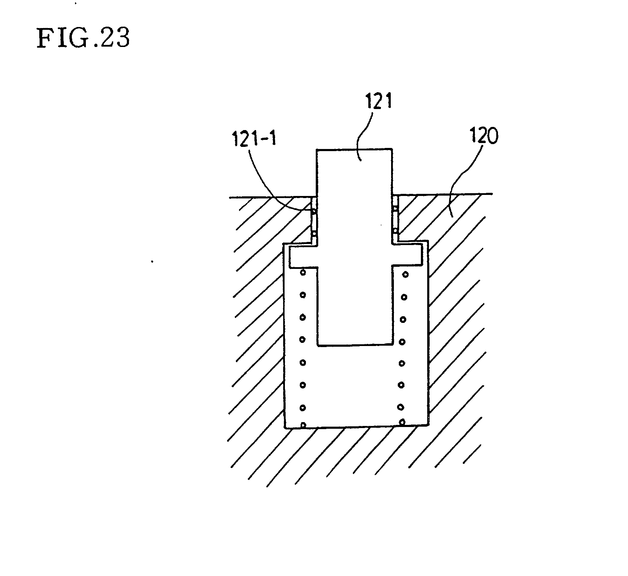

Fig. 23 is a cross-sectional view illustrating the reset button 121 installed on the housing 120. The reset button 121 is slidably fitted in a hole defined in the upper wall of the housing 120 in such a manner that it is separated from the hole. The reset button 121 is upwardly biased by a compression coil spring so that its upper end is in a state protruded from the hole of the housing 120. At least one seal packing 121-1 is fitted around the reset button 121 to provide a sealing effect between the housing 120 and reset button 121.

Now, the syringe device having the above mentioned configuration according to the present invention will be described.

First, the push plate 154 of the piston push means 150 is threadedly coupled to the rotating shaft 131 in such a manner that it is disposed at the middle portion of the rotating shaft 131. Thereafter, the engaging pin 133 is coupled to the lower end of the rotating shaft 131. Also, the cap type head 131-1 is threadedly coupled to the upper end of the rotating shaft 131. The rotating shaft 131 is then inserted into the lower end of the piston 122 until the fitting member 155 of the piston push means 150 is fitted in the lower end of the piston 122. In this state, the snap ring groove 156a of the fitting member 155 engages with the snap ring 122-4 of the piston 122. Also, the engaging protrusions 151-1 of the push plate 154 engage with optional ones of the engaging grooves 122-3 of the piston 122. The piston 122, which is coupled to the rotating shaft 131, is then fitted in the syringe 21 in such a manner that it is completely inserted into the syringe 21, as indicated by the double-dotted line in Fig. 22. In this state, a disposable injection needle (not shown) is fitted onto the tip of the syringe 21. Thereafter, the injection needle is penetrated into the interior of a phial through the plug of the phial. In this state, the piston 122 is pulled, along with the rotating shaft 131, to suck a liquid medicine contained in the phial into the syringe 21, as indicated by the solid line in Fig. 22. In order to allow the engaging pin 133 of the rotating shaft 131 to be accurately engaged in the cross groove 132-1 of the coupling member 132 when the syringe 21 filled with the liquid medicine is inserted into the syringe receiving chamber 126 of the housing 120, it is necessary to appropriately adjust an initial length of the rotating shaft 131 protruded from the piston 122 in accordance with the amount of the liquid medicine contained in the syringe 21. In order to achieve an easy and convenient adjustment of the initial protruded length of the rotating shaft 131, a scale (not shown) may be formed on the housing 120. Alternatively, a mark (not shown) indicative of a reference position for the rotating shaft 131 may be formed on the housing 120. Otherwise, a length measuring jig may be used. In this state, the syringe 21, in which the piston 122 is fitted, is inserted into the syringe receiving chamber 126 of the housing 120 in such a manner that the engaging pin 133 of the rotating shaft 131 is engaged in the cross groove 132-1 of the coupling member 132, as indicated by the arrow in Fig. 20. Thereafter, the cover 110 is threadedly coupled to the upper end of the housing 120, so that the injection needle unit is coupled to the syringe tip 21-1 of the syringe 21.

When the motor (not shown) drives under the above condition, its drive force is transmitted to the gear 132-3 via the power transmission means 130. Accordingly, the coupling member 132 integral with the gear 132-3 rotates. The rotation of the coupling member 132 results in a rotation of the rotating shaft 131 because the engaging pin 133 of the rotating shaft 131 is engaged in the cross groove 132-1 of the coupling member 132. The rotation of the rotating shaft 131 is carried out in a speed-reduced manner because the drive force of the motor is transmitted via the power transmission means 130. When the rotating shaft 131 rotates, the push means 150 moves vertically because the guide protrusion 151 of the push plate 154 is engaged in the guide groove 25 of the housing 120. For example, when the rotating shaft 131 rotates counter-clockwise, as shown in Fig. 22, the push plate 154 moves upwardly while being guided by the guide groove 25. As a result, the piston 122 coupled to the push plate 154 moves upwardly. Accordingly, the liquid medicine contained in the syringe 21 is injected into the body of the user, into which the injection needle of the injection needle unit through the connector 2 penetrates, via the connector 2 and feeding tube 1. As the injection of the liquid medicine is carried out for a prolonged time, the piston 122 reaches its initial position indicated by the double-dotted line in Fig. 22. In this state, the user separates the injection needle unit from the body and completes the use of the syringe device. Thereafter, the connector 2 of the injection needle unit is separated from the cover 110 which is, in turn, released from the housing 120. The syringe 21, piston 122, push means 150 and rotating shaft 131 assembled together are removed from the syringe receiving chamber 126 of the housing 120. Where it is desired to use again the syringe device, a liquid medicine is filled in the syringe 21 in accordance with the above mentioned piston function. Thereafter, the user rotates the rotating shaft 131 by hand so that the rotating shaft 131 is inserted into the piston 122 to its original position. That is, the rotating shaft 131 is adjusted to have a desired initial length protruded from the piston 122. In order to achieve an easy adjustment of the initial protruded length of the rotating shaft 131, it may be possible to use a scale formed on the housing 120, a mark indicative of a reference position for the rotating shaft 131 formed on the housing 120, or a length measuring jig. As mentioned above, the reason why the rotating shaft 131 is adjusted to have a desired initial length protruded from the piston 122 is to allow the engaging pin 133 of the rotating shaft 131 to be accurately engaged in the cross groove 132-1 of the coupling member 132 when the syringe 21 is fitted in the syringe receiving chamber 126 of the housing 120.

Once the push plate 154 is threadedly coupled to the rotating shaft 131, it is prevented from being separated from the rotating shaft 131 because the cap type head 131-1 is threadedly coupled to the upper end of the rotating shaft 131. Accordingly, an improvement in durability is obtained.

Where the injection of the liquid medicine contained in the syringe 21 is achieved by an upward movement of the piston 122 resulting from an upward movement of the push means 150 along the rotating shaft 131, it is necessary to return the upwardly-moved push means 150 to its initial position after filling again the syringe 21 with a liquid medicine to inject again the liquid medicine. However, it is disadvantageous to return the push means 150 to its initial position by reversely rotating the rotating shaft 131 using a drive force from the motor. This is because the drive force from the motor is transmitted to the rotating shaft 131 in a speed-reduced manner, so that a lengthened time of about 5 to 10 minutes is taken for the push means to return to its initial position. In this case, accordingly, there is a wastage of time. In order to solve this disadvantage, in accordance with the illustrated embodiment of the present invention, the rotating shaft 131 is configured to be separable from the motor so that it is manually rotated. Accordingly, it is possible to easily adjust the initial position of the push means by a manual rotation of the rotating shaft 131. The rotating shaft 131 is also configured to be rotated only in one direction by a drive force from the motor. Accordingly, the control for the motor is simplified. This results in a reduction in the manufacturing costs.

In particular, all the cover 110, battery cover 125, reset button 121 and bottom cover 140 are sealably configured in accordance with the illustrated embodiment of the present invention, even though such a configuration is omitted from the drawings because it is well known. In this case, a vacuum is generated in the interior of the housing 120 as the liquid medicine contained in the syringe 21 is injected into the body of the user. As a result, the piston 122 is overloaded. This problem may be eliminated by forming the reset button 121 using a well-known semi-permeable material preventing penetration of moisture while allowing ventilation of air. In this case, it is possible to prevent a vacuum from being generated in the interior of the housing 120 while still maintaining a moisture sealing effect between the housing 120 and reset button 121. An increase in the manufacturing costs occurs when the entire portion of the housing 120 is made of the semi-permeable material. However, where a small part of the housing 120, for example, the reset button 121, is made of the semi-permeable material, it is possible to minimize an increase in the manufacturing costs while maintaining a permeable effect for the housing 120 and providing convenience in installation. In this case, preferably, at least one seal packing 121-1 is fitted around the reset button 121 to provide a desired sealing effect between the housing 120 and reset button 121.

As apparent from the above description, the present invention provides a portable automatic syringe device having a configuration including a separable rotating shaft adapted to provide a drive force to a piston included in the automatic syringe device. The rotating shaft can be separated, along with the piston, from a housing of the syringe device upon re-filling a syringe of the syringe device with a liquid medicine. After the re-filling of the liquid medicine, the length of the rotating shaft is manually set. Accordingly, it is possible to easily adjust the initial position of the push means by a manual rotation of the rotating shaft. The rotating shaft is also configured to be rotated only in one direction by a drive force from the motor. Accordingly, the control for the motor is simplified. This results in a reduction in the manufacturing costs. It is also possible to reduce the time taken for the re-filling of the liquid medicine.

Although the automatic syringe device according to the present invention maintains a sealability by providing seal packings for various elements such as covers, plugs and buttons, at least one of those elements (for example, a reset button) is made of a semi-permeable material preventing penetration of moisture while allowing ventilation of air. In this case, it is possible to prevent a vacuum from being generated in the interior of the housing while still maintaining a moisture sealing effect between the housing and reset button. Accordingly, there is no overload in the supply of the liquid medicine.

## Claims

1. A portable automatic syringe device comprising a housing (120) having a syringe (21) separably received in a syringe receiving chamber (126), an injection needle unit (3) coupled to the housing (120) and communicating with the syringe (21), a piston (122) slidably fitted in the syringe (21) and separable received in the housing (120), the piston (122) being longitudinally movable in the syringe (21), a threaded rotating shaft (131), push means (150) threadedly coupled to the rotating shaft (131) for moving the piston (122) so that the push means (150) moves along the rotating shaft (131) when the rotating shaft (131) rotates, power transmission means (130) fixedly installed in the housing (120) for transmitting a drive force generated from power generating means (125) to the rotating shaft (131), and a coupling member (132) coupling said rotating shaft (131) to said power transmission means (130),
said syringe (21), piston (122), push means (150) and rotating shaft (131), are together slidable with respect to the housing (120) between a first position with an end of the rotating shaft (131) protruding into the coupling member (132) and a second position with the rotating shaft (131) removed from the housing (120) whereby the rotating shaft (131) can be rotated manually, the coupling member (132) and rotating shaft (131) comprising means (132-1, 133) for coupling the rotating shaft (131) to, and for decoupling the rotating shaft from, the coupling member (132), said means (132-1, 133) coupling said rotating shaft (131) to the coupling member (132) when the syringe (21), piston (122), push means (150) and rotating shaft (131) are slid into said first position, said means (132-1, 133) decoupling the rotating shaft (131) from the coupling member (132) when the syringe (21), piston (122), push means (150) and rotating shaft (131) are slid from the first position to the second position, **characterized in that**
the piston (122), the push means (150) and the rotating shaft (131) are assembled such as to form a single complete assembly, that the syringe receiving chamber (126) is formed at its inner surface with a longitudinal extending guide groove (25) and the push means (150) is provided with a radially extending guide protrusion (151) engageable in said guide groove (25) so as to guide a longitudinal movement of the push means (150) along the rotating shaft (131) and that said means (132-1, 133) for coupling the rotating shaft (131) to, and for decoupling the rotating shaft (131) from the coupling member (132) comprises a cross groove (132-1) in the coupling member (132) and a horizontal engaging pin (133) on the end of the rotating shaft (131), said horizontal engaging pin (133) engaging said cross groove (132-1) when the end of the rotating shaft (131) protrudes into the housing (120) a distance sufficient to contact the coupling member (132).

2. A portable automatic syringe as claimed in claim 1, wherein the coupling member (132) comprises a reduction gear (132-3) that engages an output gear of said power transmission means (130).

3. A portable automatic syringe as claimed in claim 1, wherein said coupling member (132) is of unitary construction with the reduction gear (132-3) being an integral part thereof.

4. A portable automatic syringe as claimed in claim 3, wherein said reduction gear (132-3) and an output gear are excentrically disposed with respect to one another, said reduction gear (132-3) engaging said power transmission means (130) along a circumference of the reduction gear (132-3) only.

5. A portable automatic syringe as claimed in claim 1, wherein the piston (122) comprises radially extending protrusions (122-2) engageable in additional guide grooves (27) longitudinally extending in the syringe receiving chamber for faciliating slidable movement of said piston (122), push means (150) and rotating shaft (131) relative to said housing (120) along said vertical axis.

## Patentansprüche

1. Tragbare automatische Spritzenvorrichtung, umfassend ein Gehäuse (120) mit einer Spritze (21), die herausnehmbar in einem Spritzenaufnahmeraum (126) aufgenommen ist, eine Injektionsnadeleinheit (3), die mit dem Gehäuse (120) verbunden ist und mit der Spritze (21) in Verbindung steht, einen Kolben (122), der verschiebbar in die Spritze (21) paßt und der herausnehmbar in dem Gehäuse (120) aufgenommen ist, wobei der Kolben (122) in Längsrichtung in der Spritze (21) verschoben werden kann, eine Gewinde-Drehwelle (131), ein Schiebemittel (150), das mit der Drehwelle (131) schraubverbunden ist, um den Kolben (122) zu bewegen, so daß das Schiebemittel (150) sich entlang der Drehwelle (131) bewegt, wenn die Drehwelle (131) sich dreht, ein Energieübertragungsmittel (130), das fest im Gehäuse (120) installiert ist, um eine Antriebskraft, die von einem Energieerzeugungsmittel (125) erzeugt wird, auf die Drehwelle (131) zu übertragen, und ein Verbindungsteil (132), das die Drehwelle (131) mit dem Energieerzeugungsmittel (130) verbindet,
wobei die Spritze (21), der Kolben (122), das Schiebemittel (150) und die Drehwelle (131) gemeinsam relativ zum Gehäuse (120) zwischen einer ersten Position, in der ein Ende der Drehwelle (131) in das Verbindungsteil (132) hineinragt, und einer zweiten Position, in der die Drehwelle (131) sich außerhalb des Gehäuses (120) befindet, verschiebbar sind, wobei die Drehwelle (131) manuell gedreht werden kann, das Verbindungsteil (132) und die Drehwelle (131) Mittel (132-1, 133) umfassen, um die Drehwelle (131) mit dem Verbindungsteil (132) zu verbinden bzw. vom Verbindungsteil (132) zu trennen, wobei die Mittel (132-1, 133) die Drehwelle (131) mit dem Verbindungsteil (132) verbinden, wenn die Spritze (21), der Kolben (122), das Schiebeteil (150) und die Drehwelle (131) in die erste Position geschoben werden, und wobei die Mittel (132-1, 133) die Drehwelle (131) vom Verbindungsteil (132) trennen, wenn die Spritze (21), der Kolben (122), das Schiebemittel (150) und die Drehwelle (131) aus der ersten Position in die zweite Position geschoben werden, **dadurch gekennzeichnet daß**
der Kolben (122), das Schiebeteil (150) und die Drehwelle (131) so zusammengebaut sind, daß sie eine komplette Einheit bilden, daß an der Innenseite des Spritzenaufnahmeraums (126) eine in Längsrichtung verlaufende Führungsnut (25) ausgebildet ist, und das Schiebeteil (150) mit einem radial verlaufenden Führungsvorsprung (151) versehen ist, der in die Führungsnut (25) eingreifen kann, um die Bewegung des Schiebeteils (150) in Längsrichtung entlang der Drehwelle (131) zu führen, und die Mittel (132-1, 133) zum Verbinden der Drehwelle (131) mit dem Verbindungsteil (132) bzw. zum Entkoppeln der Drehwelle (131) vom Verbindungsteil (132) aus einer Kreuznut (132-1) im Verbindungsteil (132) und einem horizontalen Eingriffsstift (133) am Ende der Drehwelle (131) bestehen, wobei der horizontale Eingriffsstift (133) in die Kreuznut (132-1) eingreift, wenn das Ende der Drehwelle (131) ausreichend weit in das Gehäuse (120) hineinragt, um das Verbindungsteil (132) zu berühren.

2. Tragbare automatische Spritze nach Anspruch 1, worin das Verbindungsteil (132) ein Untersetzungszahnrad (132-3) aufweist, das in ein Abtriebszahnrad des Kraftübertragungsmittels (130) eingreift.

3. Tragbare automatische Spritze nach Anspruch 1, worin das Verbindungsteil (132) einheitlich mit dem Untersetzungszahnrad (132-3) gestaltet ist, so daß es einen integralen Teil davon bildet.

4. Tragbare automatische Spritze nach Anspruch 3, worin das Untersetzungszahnrad (132-3) und ein Abtriebszahnrad exzentrisch zueinander angeordnet sind, wobei das Untersetzungszahnrad (132-3) nur entlang des Umfangs des Untersetzungszahnrads (132-3) in das Kraftübertragungsmittel (130) eingreift.

5. Tragbare automatische Spritze nach Anspruch 1, worin der Kolben (122) radial verlaufende Vorsprünge (122-2) aufweist, die in zusätzliche Führungsnuten (27) eingreifen können, die im Spritzenaufnahmeraum in Längsrichtung verlaufen, um eine Gleitbewegung des Kolbens (122), des Schiebeteils (150) und der Drehwelle (131) relativ zum Gehäuse (120) entlang der Vertikalachse zu erleichtern.

## Revendications

1. Dispositif de seringue automatique portatif comprenant un boîtier (120) ayant une seringue (21) reçue de façon séparable dans une chambre réceptrice de seringue (126), une unité aiguille d'injection (3) couplée au boîtier (120) et communiquant avec la seringue (21), un piston (122) disposé de façon coulissante dans la seringue (21) et reçu de façon séparable dans le boîtier (120), le piston (122) étant mobile longitudinalement dans la seringue (21), un arbre rotatif fileté (131), un moyen de poussée (120) couplé par filetage à l'arbre rotatif (131) pour déplacer le piston (122) de telle sorte que le moyen de poussée (150) se déplace le long de l'arbre rotatif (131) lorsque l'arbre rotatif (131) tourne, un moyen de transmission de puissance (130) monté de façon fixe dans le boîtier (120) pour transmettre une force motrice générée à partir du moyen de production d'énergie (125) vers l'arbre rotatif (131), et un élément d'accouplement (132) couplant ledit arbre rotatif (131) au dit moyen de transmission de puissance (130),
ladite seringue (21), ledit piston (122), ledit moyen de poussée (150) et ledit arbre rotatif (131) peuvent coulisser ensemble par rapport au boîtier (120) entre une première position avec une extrémité de l'arbre rotatif (131) dépassant dans l'élément d'accouplement (132) et une seconde position avec l'arbre rotatif (131) retiré du boîtier (120) moyennant quoi l'arbre rotatif (131) peut être tourné manuellement, l'élément d'accouplement (132) et l'arbre rotatif (131) comprenant un moyen (132-1, 133) pour coupler l'arbre rotatif (131) à l'élément d'accouplement (132), et pour le découpler de celui-ci, ledit moyen (132-1, 133) couplant ledit arbre rotatif (131) à l'élément d'accouplement (132) lorsque la seringue (21), le piston (122), le moyen de poussée (150) et l'arbre rotatif (131) sont glissés dans ladite première position, ledit moyen (132-1, 133) découplant l'arbre rotatif (131) de l'élément d'accouplement (132) lorsque la seringue (21), le piston (122), le moyen de poussée (150) et l'arbre rotatif (131) sont glissés de la première à la seconde position, **caractérisé en ce que**
le piston (122), le moyen de poussée (150) et l'arbre rotatif (131) sont assemblés de telle sorte qu'ils forment un seul assemblage complet, **en ce que** la chambre recevant la seringue (126) comprend au niveau de sa surface interne une rainure de guidage s'étendant longitudinalement (25) et le moyen de poussée (150) est muni d'une protubérance s'étendant radialement (151) et pouvant engrener avec ladite rainure de guidage (25) afin de guider un mouvement longitudinal du moyen de poussée (150) le long de l'arbre rotatif (131) et **en ce que** ledit moyen (132-1,133) pour coupler l'arbre rotatif (131) à l'élément d'accouplement (132), et pour le découpler de celui-ci, comprend une rainure en croix (132-1) dans l'élément d'accouplement (132) et une clavette d'accouplement horizontale (133) sur l'extrémité de l'arbre rotatif (131), ladite clavette d'accouplement horizontale (133) engrenant avec ladite rainure en croix (132-1) lorsque l'extrémité de l'arbre rotatif (131) dépasse suffisamment dans le boîtier (130) pour entrer en contact avec l'élément d'accouplement (132).

2. Seringue automatique portative selon la revendication 1, dans laquelle l'élément d'accouplement (132) comprend un engrenage de démultiplication (132-3) qui engrène avec un engrenage de sortie dudit moyen de transmission de puissance (130).

3. Seringue automatique portative selon la revendication 1, dans laquelle ledit élément d'accouplement (132) forme un ensemble monobloc avec l'engrenage de démultiplication (132-3) étant solidaire de celui-ci.

4. Seringue automatique portative selon la revendication 3, dans laquelle ledit engrenage de démultiplication (132-3) et un engrenage de sortie sont disposés de façon excentrée l'un par rapport à l'autre, ledit engrenage de démultiplication (132-3) engrenant avec ledit moyen de transmission de puissance (130) le long de la circonférence de l'engrenage de démultiplication (132-3) uniquement.

5. Seringue automatique portative selon la revendication 1, dans laquelle le piston (122) comprend des protubérances s'étendant radialement (122-2) pouvant engrener avec d'autres rainures de guidage (27) s'étendant longitudinalement dans la chambre réceptrice de seringue pour faciliter le mouvement coulissant dudit piston (122), dudit moyen de poussée (150) et dudit arbre rotatif (131) par rapport au dit boîtier (120) le long dudit axe vertical.
